# EUROPEAN PATENT APPLICATION

(11) **EP 2 487 285 A1**
(43) Date of publication of application: **15.08.2012**
(21) Application number: 10821851.2
(22) Date of filing: 17.09.2010
(51) Int. Cl.: D04H 1/54, A61F 13/15, A61F 13/49, A61F 13/511

(54) **NONWOVEN FABRIC**

(30) Priority: 09.10.2009 JP 2009235513
(71) Applicant: Unicharm Corporation, Ehime 799-0111 (JP)
(72) Inventor: MIZUTANI, Satoshi, Kanonji-shi Kagawa 769-1602 (JP); GODA, Hiroki, Kanonji-shi Kagawa 769-1602 (JP); ISHIKAWA, Hideyuki, Kanonji-shi Kagawa 769-1602 (JP); OBA, Toru, Kanonji-shi Kagawa 769-1602 (JP)
(74) Representative: Knights, Rupert
(86) International application number: PCT/JP2010/066134
(87) International publication number: WO 2011/043180

(57) **Abstract**

A nonwoven fabric alternately formed on its upper surface with crests and troughs extending in parallel to one another in one direction to prevent bodily fluids from staying in the crests. Short fibers 11 of thermoplastic synthetic resin are fusion bonded to one another to form a nonwoven fabric 1. The nonwoven fabric 1 is formed on its upper surface 2 with crests 6 and troughs 7 extending in parallel to one another. The crests 6 include first crests 6a having a uniform height dimension from a lower surface 3 of the nonwoven fabric 1 and second crests 6b having a uniform height dimension from a lower surface 3 of the nonwoven fabric 1 which is smaller than the height dimension of the first crests 6a. Density of the nonwoven fabric 1 gradually increases in the order of the first crests 6a, the second crests 6b and the troughs 7. The first crests 6a are formed so that the density of the first crests 6a remains lower than the density of the second crests 6b even when the first crests 6a are compressed toward the lower surface 3 until the first crests 6a becomes flush with the level of the second crests 6b.

## Description

### {Technical Field}

The present invention relates to nonwoven fabrics formed of short fibers of thermoplastic synthetic resin.

It is conventionally known to fusion bond short fibers of thermoplastic synthetic resin together and thereby to obtain nonwoven fabrics. As an example of such nonwoven fabrics, JP 2008-25080 A (PTL 1) discloses a nonwoven fabric having ridges and grooves extending in parallel to one another in a machine direction wherein these ridges and grooves alternate in a cross direction extending orthogonally to the machine direction.

### {Citation List}

### {Patent Literature}

{PTL 1} JP 2008-25080 A

### {Summary}

### {Technical Problem}

The ridges in the nonwoven fabric disclosed in PTL 1 include the relatively high ridges and the relatively low ridges. The relatively high ridges and the relatively low ridge are formed to be the same in their basis mass wherein the basis mass of both these ridges having different height dimensions in the middle segments thereof in the width direction than the basis mass of the grooves. Assuming that such a nonwoven fabric is used as the liquid-pervious inner sheet in the wearing article such as a disposable diaper or a menstruation napkin and comes in tight contact with the wearer's skin, the relatively high ridges are primarily compressed. In the inner sheet at this moment, the compressed ridges have the density thereof further increased and, in consequence, bodily fluids are apt to stay in these ridges and barred from smoothly moving toward the surrounding region of the lower density. When the ridges are compressed, for example, to the same level with the relatively low ridges, the ridges having been compressed in this manner will locally pressed against the wearer's skin. Eventually, the inner sheet as a whole may not come in soft and close contact with the wearer's skin and may have an uncomfortable texture.

An object of this invention is to provide a nonwoven fabric improved so that even when the nonwoven fabric is formed on its upper surface with the ridges and the grooves, bodily fluids excreted onto the upper surface may not stay in these ridges after this upper surface has come in close contact with the wearer's skin.

### {Solution to Problem}

According to the present invention, there is provided a nonwoven fabric formed of short fibers of thermoplastic synthetic resin fusion bonded to one another and having a length direction, a width direction and a thickness direction extending orthogonally one to another, including an upper surface and a lower surface opposed to the upper surface as viewed in the thickness direction wherein
the upper surface is formed with crests and troughs undulating in the width direction and extending in parallel to one another in the length direction and
the crests include first crests having a uniform height dimension and second crests having a uniform height dimension measured from the lower surface wherein the height dimension of the first crests is larger than the height dimension of the second crests.

The present invention resides in that a density of the nonwoven fabric gradually increases in order of the first crests, the second crests and the troughs and the density of the first crests remains lower than the density of the second crests even when the first crests are compressed from the upper surface toward the lower surface until the first crests become flush with the second crests.

According to one embodiment of the present invention, the nonwoven fabric has a basis weight in a range of 18 to 100g/m² and each of the short fibers has a fineness in a range of 1 to 8dtex and a fiber length in a range of 20 to 80mm, and the nonwoven fabric has been modified to become hydrophilic.

According to another embodiment of the present invention, the short fibers are conjugate fibers including two types of the thermoplastic synthetic resin having different fusion temperatures and these two types of the thermoplastic synthetic resin are fusion bonded to each other via one of the thermoplastic synthetic resins having a lower fusion temperature.

According to still another embodiment of the present invention, the height dimension of the first crests is in a range of 1 to 5mm and the height dimension of the second crests is lower than the height dimension of the first crests by a range of 0.5 to 2mm.

According to yet another embodiment of the present invention, the first crests and the second crests are formed alternately in the width direction and each of the troughs is interposed between each pair of the adjacent first crest and second crest.

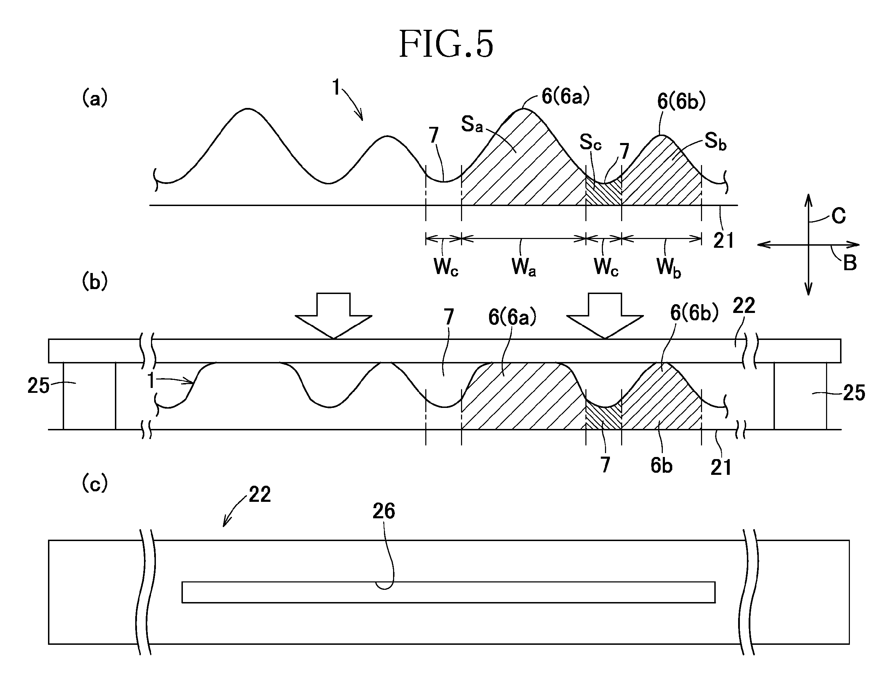

A measuring method used for measuring "density of nonwoven fabric" will be described later with reference to Figs. 5 through 7.

### {Advantageous Effects of Invention}

The nonwoven fabric according to the present invention has the crests and the troughs extending in parallel to one another in the length direction wherein the crests include the first crests each having a uniform height dimension and the second crests each having a uniform height dimension which is smaller than the height dimension of the first crests wherein both of the height dimensions are defined by a dimension of the nonwoven fabric in the thickness direction and these crests cooperate with the troughs to form the upper surface of the nonwoven fabric with the ridges and the grooves. The density of the nonwoven fabric gradually increases in the order of the first crest, the second crest and the trough wherein the density of the first crest is maintained lower than the density of the second crest even when the first crest is compressed to the same level as the second crest. On the assumption that such a nonwoven fabric is used as the inner sheet of the wearing article, even when the upper surface of the nonwoven fabric comes in close contact with the wearer's skin and the first crests having initially been highest are compressed to the same level as the second crests, the density gradient of the first crests, the second crests and the troughs is maintained. In principle, bodily fluids excreted onto the inner sheet are apt to flow from the region having the relatively low density toward the region having the relatively high density, specifically, from the first crests to the second crests, then from the second crest to the troughs. The stabilized density gradient assures that bodily fluids smoothly flow toward the troughs without staying in the first crests and the wearer is free from discomfort feeling of wetness even if the first crests directly come in contact with the wearer's skin. In addition, the first crests would not locally press against the wearer's skin since the density of the first crests remains lower than that of the second crests. In other words, the nonwoven fabric according to the present invention has a uniform texture.

### {Brief Description of Drawings}

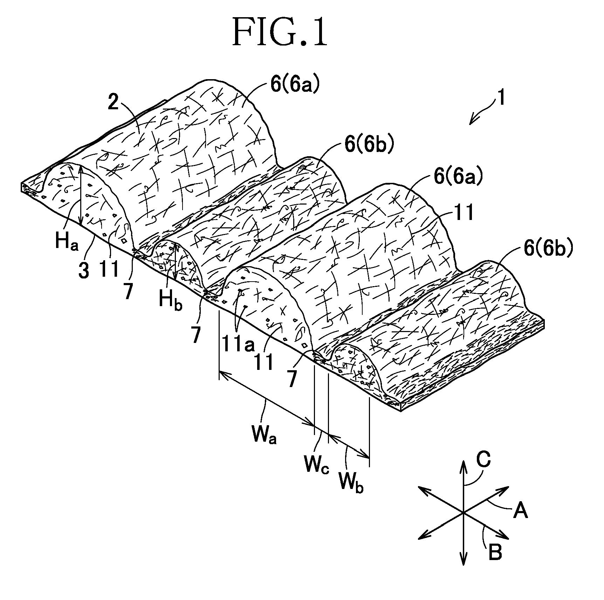

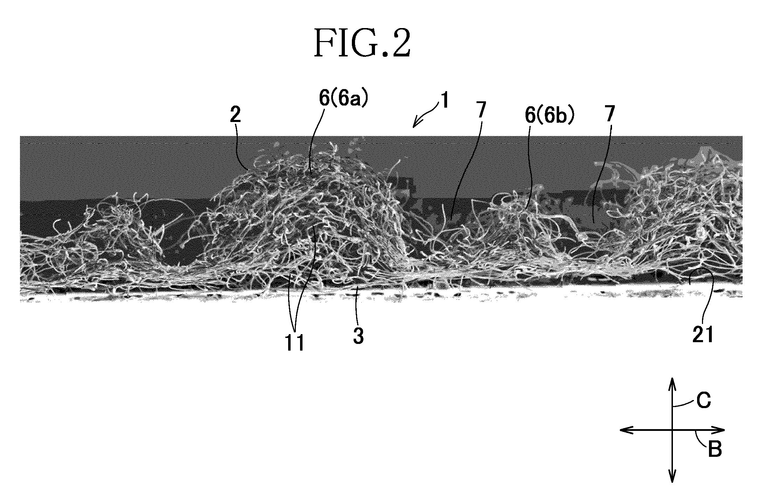

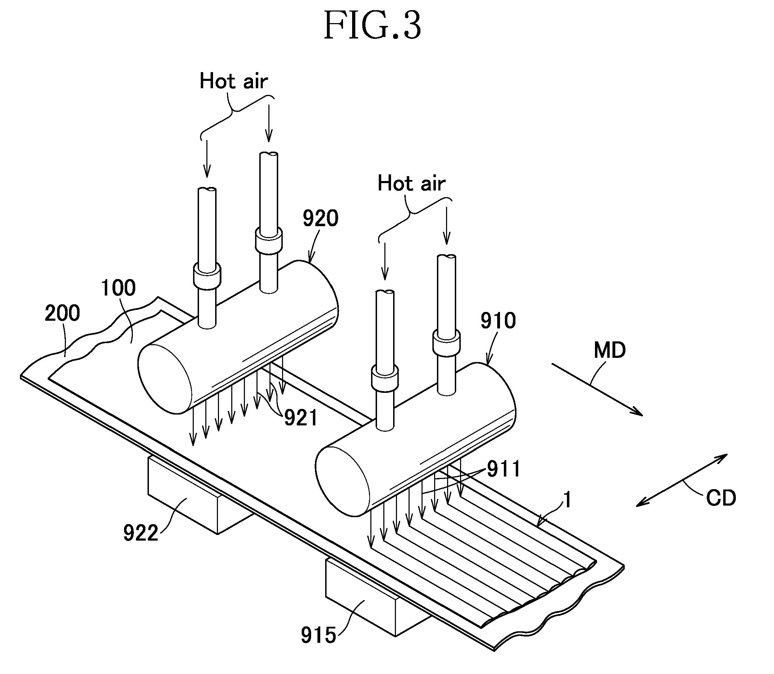

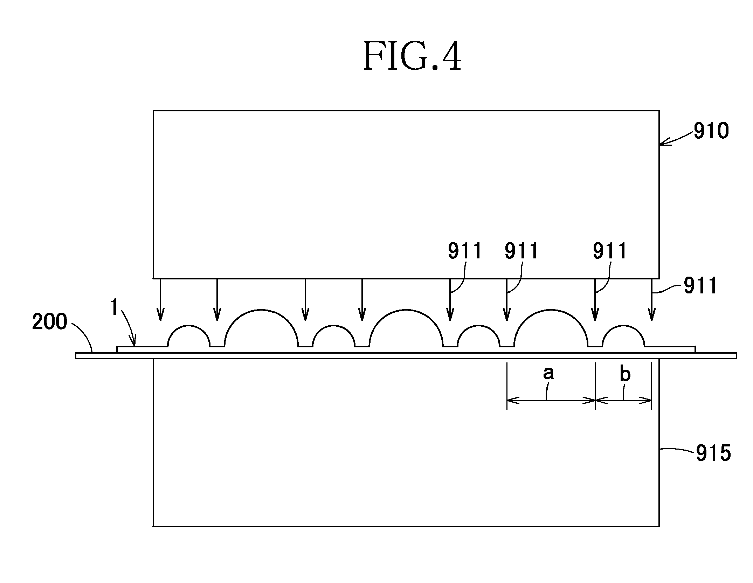

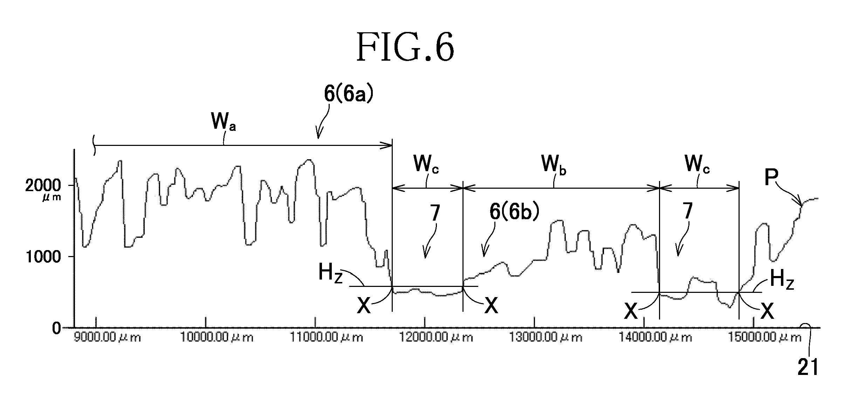

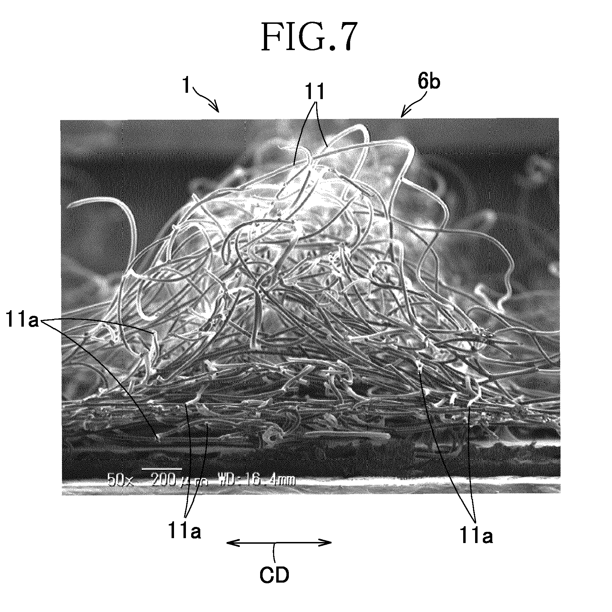

{Fig. 1} Fig. 1 is a partial perspective view of a nonwoven fabric according to the present invention.
{Fig. 2} Fig. 2 is a photograph of a section of the nonwoven fabric in a cross direction.
{Fig. 3} Fig. 3 is a diagram partially illustrating a process for manufacturing the nonwoven fabric.
{Fig. 4} Fig. 4 is a schematic front elevation view showing a first array of air jet nozzles.
{Fig. 5} Fig. 5 is a diagram illustrating how the section of the nonwoven fabric is transformed.
{Fig. 6} Fig. 6 is a graphic diagram plotting the surface contour of the nonwoven fabric based on observation.
{Fig. 7} Fig. 7 is a photograph showing a cross-section of fibers.

### {Description of Embodiments}

Details of nonwoven fabrics according to the present invention will be more fully understood from the description given hereunder with reference to the accompanying drawings.

Fig. 1 is a partial perspective view of a nonwoven fabric 1 according to the present invention and Fig. 2 is a 50-fold magnified photograph exemplarily showing cross-section of the nonwoven fabric. It should be noted here that the photograph of Fig. 2 is really an assembly including a plurality of photographs joined one with another in a width direction since it is impossible to record the 50-fold magnified photograph of the nonwoven fabric over its sufficiently wide range in a single shot. The nonwoven fabric 1 has a length direction, a width direction and a thickness direction extending orthogonally one to another and designated by the double-headed arrows A, B and C, respectively. The nonwoven fabric 1 has an upper surface 2 and a lower surface 3 opposed to each other in the thickness direction C. The upper surface 2 is formed with crests 6 and troughs 7 extending in parallel to one another in the length direction A and undulating in the width direction B. The crests 6 include first crests 6a having a height Hₐ and second crests 6b having a height H_{b} in the thickness direction C. The height Hₐ of the first crests 6a is larger than the height H_{b} of the second crests 6b. These first crests 6a and second crests 6b are arranged alternately in the width dimension B and between each pair of the adjacent first and second crests 6a, 6b, each of the troughs 7 is interposed.

Such nonwoven fabric 1 is formed by subjecting short fibers (staples) 11 of thermoplastic synthetic resin preferably having a fineness in a range of 1 to 8dtex and a fiber length in a range of 20 to 80mm to blasts of hot air and thereby fusion bonding them to one another. The nonwoven fabric 1 is suitable for use as a liquid-pervious inner sheet in a bodily fluid-absorbent wearing article such as disposable diaper or sanitary napkin and, for such intended use, the nonwoven fabric 1 preferably has a basis mass in a range of 18 to 100g/m² and preferably has been previously treated to become hydrophilic. The nonwoven fabric 1 used as the inner sheet includes a lower surface 3 being substantially flat and an upper surface 2 formed with troughs 7 defining grooves each having a dimension W_{c} in the width direction B in a range of 0.4 to 2mm and first crests 6a defining ridges each having a dimension Wₐ in the width dimension B in a range of 2 to 5mm, which is larger than a dimension W_{b} of second crests 6b in the width dimension B. Preferably, a height Hₐ of the first crests 6a is in a range of 1 to 5mm, a height H_{b} of the second crests 6b is lower than Hₐ by a range of 0.5 to 2mm and a level of the troughs 7 is lower than the height Hₐ by a range of 0.7 to 2.5mm. To facilitate the short fibers 11 to be fusion bonded to one another and to make the nonwoven fabric 1 elastically compressible in the thickness direction C, each of the short fibers 11 is preferably prepared in the form of a conjugate fiber made of two types of synthetic resin having different fusion temperatures. Such conjugate fibers may be fusion bonded to one another by fusing the component fibers of which fusion temperature is lower than the other. Combination of these different types of component synthetic resin includes, for example, polyethylene/polyester or polyethylene/polypropylene. The conjugate fiber may be of core-in-sheath type and side-by-side type. The core-in-sheath type conjugate fiber may be concentric core type or eccentric core type.

Fig. 3 is a diagram partially illustrating a process for manufacturing the nonwoven fabric 1 and Fig. 4 is a schematic front elevation view showing a first array of air jet nozzles 910 used in the process of Fig. 3. Referring to Fig. 3, a conveyor belt 200 which is air-permeable in the thickness direction is loaded with a carded web 100 formed of the short fibers 11 and runs in a machine direction MD. The carded web 100 is subjected to blasts of a plurality of hot air jets 921 ejected from a second air jet nozzle array 920 and the air jet 921 is sucked by a suction box 922 through the belt 200. The air jet 921 has a temperature not higher than a level to soften the short fibers 11 and serves to compress the carded web 100 to a range of 2/3 to 1/4 of its initial thickness and thereby to stabilize a texture of the carded web 100. Then the carded web 100 is subjected to blasts of a plurality of heated air jets 911 ejected from a first air jet nozzle array 910. The air jets 911 has an airflow rate, a pressure and a temperature adjusted so that the short fibers 11 may be moved in a cross direction CD orthogonal to the machine direction MD and may be fusion bonded to one another.

Referring to Fig. 4, the first air jet nozzle array 910 includes a plurality of nozzles (not shown) arranged at predetermined pitches a and b in the cross direction CD, from which the air jets 911 are ejected toward the carded web 100. These air jets 911 are sucked by a suction box 915 through the belt 200. The positions of the air jets 911 arranged at the pitches a and b in the cross direction CD correspond to the positions of the troughs 7 formed in the nonwoven fabric 1 as shown in Fig. 1. In the carded web 100, the short fibers 11 located just below the air jets 911 are partially shared half-and-half on both sides about the cross direction CD, respectively, and these portions shared half-and-half participate in formation of the first crests 6a and the second crests 6b while the portions staying immediately below the air jets 911 form the respective troughs 7. The short fibers 11 lying between each pair of the adjacent air jets 911 are moved in the cross direction CD to form the first crest 6a or the second crest 6b. Specifically, the short fibers 11 lying between each pair of the adjacent air jets 911 arranged at the relatively large pitch a move to form the first crest 6a and the short fibers 11 lying between each pair of the adjacent air jets 911 arranged at the relatively small pitch b move to form the second crest 6b. The first crests 6a formed concurrently with the troughs 7 in this manner have a height larger than a height of the second crests 6b and a density lower than a density of the second crests 6b. In the troughs 7 formed immediately below the respective air jets 911, the short fibers 11 are compressed in the thickness direction C and densified.

When manufacturing the nonwoven fabric 1 by using the process illustrated in Figs. 3 and 4, assuming that the carded web 100, for example, having a basis mass in a range of 30 to 50g/m² is conveyed in the machine direction MD at a moderate speed, for example, at a speed in the order of 10m/min, an ejection quantity of the air jets 911 from the first air jet nozzle array 910 may be set to a low level and the second air jet nozzle array 920 may be eliminated. On the assumption that such carded web 100 is conveyed at a speed in a range of 30 to 40m/min, the second air jet nozzle array 920 may be used preferably in combination with the first air jet nozzle array 910 to stabilize the carded web 100 in advance.

In the nonwoven fabric 1 of Fig. 1 obtained in this manner, a density of the first crests 6a may be adjusted to be lower than a density of the second crests 6b and a density of the second crests 6b may be adjusted to be lower than a density of the troughs 7 to achieve a desirable behavior of the nonwoven fabric 1 when such a nonwoven fabric is used as the liquid-absorbent inner sheet of the bodily fluid-absorbent wearing article. Specifically, bodily fluids excreted on the upper surface 2 of the nonwoven fabric 1 move from the region having a low density toward the region having a high density. In other words, bodily fluids smoothly move from the first crests 6a toward the troughs 7 and from the second crests 6b toward the troughs 7 in accordance with the density gradient of the nonwoven fabric 1. When the nonwoven fabric 1 is bent and, in consequence, the first crests 6a and the second crests 6b move closer to each other, bodily fluids move from the first crests 6a toward the second crests 6b. Therefore, certain amount of bodily fluids would not stay in the first crests 6a being first to come in contact with the wearer's skin and would not create a discomfort feeling of wetness against the wearer. In addition, even when the nonwoven fabric 1 is compressed until the first crests 6a are compressed to the same level with the second crests 6b, the density of the first crests 6a remains lower than the density of the second crests 6b. Therefore, even in such a situation, the first crests 6a would not create a discomfort feeling of wetness against the wearer. Also, even when the first crests 6a are compressed together with the second crests 6b, the first crests 6a subjected to a higher degree of compression than that to which the second crests 6b are subjected would not be pressed against the wearer's skin more tightly than the second crests 6b, since the density of the first crests 6a is maintained lower than that of the second crests 6b. Consequently, the texture of the nonwoven fabric would not become uneven and create a discomfort feeling to wear against the wearer.

TABLE 1 indicates primary manufacturing conditions and evaluation results with respect to the nonwoven fabric 1 as one example of the present invention together with those with respect to comparative examples.

The nonwoven fabric according to the embodiment indicated in TABLE 1 was manufactured in the equipment exemplarily illustrated in Figs. 3 and 4 without using the second air jet nozzle array 920 under the primary conditions as will be described below. In this embodiment, the running speed of the carded web 100 was sufficiently low to save use of the second air jet nozzle array 920.
(1) Short fiber (staple): core-in-sheath type conjugate fiber having fineness in a range of 2.6 to 3.3dtex, a fiber length in a range of 38 to 51mm, polyester as the core component and polyethylene as the sheath component.
(2) Carded web: basis mass of 35g/m² and running speed of 10m/min in the machine direction.
(3) First air jet nozzle array: nozzle diameter of 1mm, nozzle pitch a = 4mm, nozzle pitch b = 2.5mm, distance between nozzle and conveyor belt of 5mm, air jet pressure of 0.06MPa and air temperature of 140°C.

The nonwoven fabric indicated in TABLE 1 as Comparative Example 1 was obtained in the same manufacturing conditions as those for the Example according to the present invention except the nozzle pitches, i.e., set to a = 4mm and b = 4mm. The nonwoven fabric according to this Comparative Example 1 is similar to the Example according to the present invention in that the crests and the troughs arranged alternately in the cross direction CD but the height of the crests is uniform and there is no discrimination between the first crests and the second crests.

The nonwoven fabric indicated in TABLE 1 as Comparative Example 2 was obtained in the same manufacturing conditions as those for the Example according to the present invention except the nozzle pitches, i.e., set to a = 3.5mm and b = 3mm. The nonwoven fabric according to this Comparative Example 2 is similar to the Example according to the present invention in that the crests and the troughs arranged alternately in the cross direction CD and the crests include the first crests and the second crests having a smaller height than a height of the first crests. However, there is not a substantial difference in the height between the first crests and the second crests.

**{TABLE 1}**

| | | | **Example** | | | **Comparative Example 1** | | | **Comparative Example 2** | | |
|---|---|---|---|---|---|---|---|---|---|---|---|
| | | | 1 st Crest | 2nd Crest | Trough | Crest | Trough | | 1st Crest | 2nd Crest | Trough |
| **Non-Compressed state** | **Height** | (mm) | 2.43 | 1.45 | — | 2.07 | — | | 1.91 | 1.53 | — |
| | **Width** | (mm) | 3.81 | 2.08 | 0.56 | 3.52 | 0.44 | | 2.82 | 2.17 | 0.62 |
| | **Sectional area** | (mm²) | 5.71 | 1.78 | 0.16 | 4.03 | 0.13 | | 3.31 | 2.13 | 0.17 |
| | **Number of fiber's cross-sections** | | 248.3 | 147.3 | 25.3 | 230.7 | 23 | | 220 | 179.3 | 28 |
| | **Fiber's density index** | (number/mm²) | 43.5 | 82.8 | 158.1 | 57.2 | 176.9 | | 66.5 | 84.2 | 164.7 |
| **Compressed state** | **Height** | (mm) | 1.52 | 1.50 | — | 1.52 | — | | 1.53 | 1.55 | — |
| | **width** | (mm) | 3.80 | 1.99 | 0.56 | 3.48 | 0.46 | | 2.77 | 2.19 | 0.64 |
| | **Sectional area** | (mm²) | 5.07 | 1.73 | 0.16 | 3.59 | 0.16 | | 1.77 | 2.10 | 0.15 |
| | **Number of fiber's cross-sections** | | 248.3 | 147.3 | 25.3 | 230.7 | 23 | | 220 | 179.3 | 28 |
| | **Fiber's density index** | (number/mm²) | 49.0 | 85.1 | 158.1 | 64.3 | 143.8 | | 124.3 | 85.4 | 186.7 |
| **Strike-through value (sec)** | | | 1.61 | | | 2.16 | | | 1.96 | | |
| **Q-Max value** (J/cm²•sec) | **Load of** | 10g/cm² | 0.178 | | | 0.373 | | | 0.366 | | |
| | **Load of** | 30g/cm² | 0.819 | | | 1.027 | | | 0.863 | | |

(a) and (b) of Fig. 5 schematically illustrating cross-sectional shape of the nonwoven fabric as the example of the present invention taken along in the cross direction CD and (c) of Fig. 5 is a plan view of a pressure plate 22 used in the step (b) of Fig. 5. In (a) of Fig. 5, the nonwoven fabric 1 is placed on a horizontal surface 21 (See Fig. 2 also) in a state free from any external force, i.e., in a non-compressed state wherein this nonwoven fabric 1 is formed with the first crests 6a, the second crests 6b and the troughs 7. In (b) of Fig. 5, the nonwoven fabric 1 was compressed by the pressure plate 22 from above until the first crests 6a are deformed to be substantially flush with the second crests 6b. The pressure plate 22 was supported by a pair of supporters 25 having a height substantially the same as the height of the second crests 6b. In the example of the present invention wherein the first crests 6a have a height of 2.43mm and the second crests 6b have a height of 1.45mm, the nonwoven fabric was compressed until the height of the first crests 6a is reduced to 1.45mm. The first crest 6a, the second crest 6b and the trough 7 respectively have widths Wₐ, W_{b} and W_{c} and cross-sectional areas Sₐ, S_{b} and S_{c} respectively indicated by shaded portions. A method to measure these cross-sectional areas will be described later with reference to Fig. 6 and the measurement result was indicated in TABLE 1.

In the nonwoven fabric as the comparative example 1, the nonwoven fabric in the non-compressed state was compressed by using the pressure plate 22 shown in Fig. 5 until a height of the crests is reduced to 1.52mm. Also in the nonwoven fabric as the comparative example 1, cross-sectional areas of the crest and the trough in the non-compressed state as well as in the compressed state were measured and the measurement result was indicated in TABLE 1.

In the nonwoven fabric as the comparative example 2, the nonwoven fabric in the non-compressed state was compressed by using the pressure plate 22 until a height of the first crests which is relatively large in the non-compressed state is reduced to a height of the second crests in the non-compressed state, i.e., 1.52mm. Also in the nonwoven fabric as the comparative example 2, cross-sectional areas of the first crest and the second crest in the non-compressed state as well as in the compressed state were measured and the measurement result was indicated in TABLE 1.

Fig. 6 is a graphic diagram plotting the cross-sectional areas Sₐ, S_{b} and S_{c} of each of the first crests 6a, the second crests 6b and the troughs 7 illustrated in Fig. 5, respectively, measured by 3D measuring device wherein the first crest 6a was partially illustrated. The nonwoven fabric 1 as an object to be measured has its lower surface 3 placed on the horizontal surface 21 and undulation of the upper surface as viewed in a cross-section taken along the cross direction CD was recorded by an outline P. As 3D measuring device, High-Accuracy Geometry Measuring System (inclusive of High-Accuracy Stage: KS-1100) and High-Speed and High-Accuracy CCD-Laser Displacement Gauge inclusive of Controller: LK-G3000V Set and Sensor Head: LK-G30) manufactured by Keyence Corporation were used and measuring conditions were set as follows:
(Stage: KS-1100)
Range of measurement: 3000µm x 30000µm
Measuring pitch: 20µm
Running speed: 7500µm/sec
(Measuring Head: LK-G3000V)
Measurement mode: Object to be measured
Installation mode: Diffuse reflection
Filtering: 4 times in average
Sampling period: 200µm

The measurement record obtained by the 3D measuring device was processed by Configuration Analysis System KS-H1A (manufactured by Keyence Corporation) to determine heights of the crests, widths of the crests and the troughs and cross-sectional areas of the crests and the troughs. Referring to Fig. 6, respective widths Wₐ, W_{b} and W_{c} of the first crest 6a, the second crest 6b and the trough 7 were determined by a method as follows. Referring to Fig. 6, an intersection point X at which an arbitrary horizontal line H_{z} extending in parallel to the horizontal surface 21 intersects with the outline P extending upward from below the horizontal line H_{z} is obtained and, if a segment of the outline segment P extending between a pair of the adjacent intersecting points X lies below the horizontal line H_{z} and a distance between these two intersecting points X is in a range of 0.4 to 2mm, this segment extending between these intersecting points X was defined as the trough 7. A region extending between each pair of the adjacent troughs 7 is defined as the crest 6 and, of the adjacent two crests 6, the crest 6 in which a top position of the outline P is relatively high was defined as the first crest 6a and the crest 6 in which a top position of the outline P is relatively low was defined as the second crest 6b. It should be noted here that each pair of the adjacent intersecting points X spaced from each other by a distance less than 0.4mm was ignored and each pair of the adjacent intersecting points X spaced from each other by a distance of 0.4mm or more was searched. The distance by which each pair of the adjacent intersecting points X are spaced from each other corresponds to the width W_{c} of the trough 7. In the process for manufacturing the nonwoven fabric 1 according to the present invention from the carded web 100, it is not preferable for the present invention to adopt a trough having the width smaller than 0.4mm because it will be difficult to form the first crest 6a and the second crest 6b if the width W_{c} of the trough 7 is narrower than 0.4mm. Also when the width W_{c} exceeds 2mm, it will be difficult to manufacture the nonwoven fabric 1 according to the present invention and, if the nonwoven fabric including the troughs each having such width W_{c} is used as the inner sheet of the bodily fluid-absorbent article, the distance between each pair of the adjacent crests 6 will be too large to assure a desired texture. For this reason, it is not preferable to adopt such excessively wide troughs 7.

With respect also to the nonwoven fabric 1 compressed by the pressure plate 22 until the first crests 6a are compressed downward to the state as illustrated in (b) of Fig. 5, the outline P was plotted by the procedure illustrated in Fig. 6 and thereby the heights Hₐ, H_{b}, the widths Wₐ, W_{b}, W_{c} and the cross-sectional areas Sₐ, S_{b} and S_{c} were measured. Referring to (c) of Fig. 5, the pressure plate 22 is formed in its middle region as viewed in the width direction with a slit 26 extending through the pressure plate 22 in its thickness direction. This slit 26 allows the 3D measuring device to be operated so that the sensor head of the laser displacement gauge in the 3D measuring device may observe the crests 6 and the troughs 7 of the nonwoven fabric 1 in the course of measuring the heights Hₐ, H_{b} and the width Wₐ, W_{b}, W_{c} in the nonwoven fabric 1 in the compressed state. The result obtained from the measurement having been carried out in this manner is recorded in TABLE 1. Measurement of the height, the width and the cross-sectional area was carried out also on the nonwoven fabric as comparative examples 1 and 2 in the same manner as in the nonwoven fabric 1 according to the present invention. The result of these measurements is also recorded in TABLE 1.

Fig. 7 is a 50-fold magnified photograph exemplarily showing a cross-section of the second crest 6b cut in the cross direction CD. The cut surface was obtained by cutting the nonwoven fabric 1 in the cross direction CD using a sharp cutter, for example, the substitute edge H-100 for KOKUYO Cutter Knife HA-B (trade name). This cut surface was photographed at 50-fold magnifications by using Real Surface View-VE-7800 manufactured by Keyence Corporation. Cross-sections 11a of the short fibers 11 appear in this photograph of the cross-section obtained in this manner. According to the present invention, the number of the cross-sections 11a (i.e., the number of fiber cross-sections) was determined with respect to the first crests 6a, the second crests 6b and the troughs 7, then respective ratios between these numbers and the cross-sectional areas of the first crests 6a, the second crests 6b and the troughs 7 were determined and the respective values of these ratios were defined as the respective fiber densities of the first crests 6a, the second crests 6b and the troughs 7 in the compressed state and in the non-compressed state. In this invention, these fiber densities are referred to also as fiber density indices. TABLE 1 indicates the fiber density indices in the example of the present invention and the comparative examples 1 and 2. As will be apparent from TABLE 1, the fiber density indices of the nonwoven fabric in the example of the present invention, the fiber density indices of the nonwoven fabric in the non-compressed state gradually increased in the order of the first crests 6a, the second crests 6b and the troughs 7. Such gradient of the fiber density indices was not changed in the compressed state also. Specifically, in the nonwoven fabric according to the present invention, even when the first crests 6a are compressed to the same level as the second crests 6b, the fiber density of the first crests 6a remains lower than that of the second crests 6b and therefore bodily fluids excreted on the upper surface 2 of the nonwoven fabric 1 may smoothly move from the first crests 6a having a relatively low density to the second crests 6b and the troughs 7 having a relatively high density.

When such a nonwoven fabric 1 is used as an inner sheet of an absorbent article of such as, for example, a disposable diaper, the nonwoven fabric 1 allows bodily fluids to move quickly from the upper surface 2 toward the lower surface 3 and this capacity can be represented, for example, by the strike-through value or Q-Max value.

The strike-through value used herein is represented by a time (unit: sec) required for 10ml of artificial urine to pass through the nonwoven fabric in the form of a test piece for measurement and the smaller the strike-through value, the sooner the permeation. To measure the strike-through value, the tester LISTER manufactured by Lenzing Technik Corporation was used. Specifically, the measuring probe was placed on the nonwoven fabric and the tester was operated in accordance with EDANA-ERT Section 150.3 liquid strike-though time method prescribed for this tester. Under the nonwoven fabric, 20 sheets of filter paper (Qualitative filter paper No. 2 manufactured by ADVANTEC MFS., INC.) were stacked in substitution for the absorbent article. The artificial urine was prepared by dissolving 200g of urea, 80g of sodium chloride, 8g of magnesium sulfate, 3g of calcium chloride and 1g of blue pigment No. 1 in 10 liter of ion-exchange water and 72mN/m of this artificial urine was used at a temperature of 20°C. Result of measurement was indicated in TABLE 1. The strike-through values measured on the comparative examples 1 and 2 were also indicated in TABLE 1.

The Q-Max value corresponds to a quantified heat quantity drawn from the wearer's skin by the inner sheet when the inner sheet wetted with bodily fluids comes in contact with the wearer's skin represented by unit of J/cm²*sec. The higher the Q-Max value of the inner sheet is, the larger the heat quantity drawn from the wearer's skin becomes and, in consequence, the wearer experiences an abrupt cold sensation. To measure the Q-Max value, KES-F7-THERMOLABO II Model high-accuracy and high-speed thermal property measuring device manufactured by KATO TECH CO., LTD. was used. As the measuring conditions, a temperature of the probe was set to <room temperature + 10°C, Q-Max measurement (cool sensitivity measurement)> and as the surface temperature of the probe, two standard levels of 10g/cm² and 30g/cm² were adopted. As the nonwoven fabric for measurement, 10 x 10 cm was used and this nonwoven fabric was placed on 3 sheets of qualitative filter paper No. 2 stacked, a cylinder having an inner diameter of 20mm was placed on the nonwoven fabric and 10cc of the artificial urine was poured into this cylinder about 5 sec, then the cylinder was removed, 20 sec after the artificial urine had been poured, the probe was put in contact with the surface of the nonwoven fabric to measure a heat transfer from the probe to the surface of the nonwoven fabric. Result of measurement was indicated in TABLE 1. As will be apparent from comparison of the example of the invention with the comparative examples, the nonwoven fabric 1 free from possibility that bodily fluids might stay on the surface 2 exhibited a relatively small Q-Max value.

### {Reference Signs List}

- 1: nonwoven fabric
- 2: upper surface
- 3: lower surface
- 6: crest
- 6a: crest (first crest)
- 6b: crest (second crest)
- 7: troughs
- 11: short fibers
- A: length direction
- B: width direction
- C: thickness direction

## Claims

1. A nonwoven fabric formed of short fibers of thermoplastic synthetic resin fusion bonded to one another and having a length direction, a width direction and a thickness direction extending orthogonally to one another, including an upper surface and a lower surface opposed to the upper surface as viewed in the thickness direction wherein
the upper surface is formed with crests and troughs undulating in the width direction and extending in parallel to one another in the length direction and
the crests comprise first crests having a uniform height dimension and second crests having a uniform height dimension measured from the lower surface wherein the height dimension of the first crests is larger than the height dimension of the second crests, wherein:
density of the nonwoven fabric gradually increases in order of the first crests, the second crests and the troughs and the density of the first crests remains lower than the density of the second crests even when the first crests are compressed from the upper surface toward the lower surface until the first crests become flush with the second crests.

2. The nonwoven fabric defined by Claim 1, wherein the nonwoven fabric has a basis weight in a range of 18 to 100g/m² and each of the short fibers has a fineness in a range of 1 to 8dtex and a fiber length in a range of 20 to 80mm, and wherein the nonwoven fabric has been modified to become hydrophilic.

3. The nonwoven fabric defined by Claim 1 or 2, wherein the short fibers are conjugate fibers comprising two types of the thermoplastic synthetic resin having different fusion temperatures and these two types of the thermoplastic synthetic resin are fusion bonded to each other via one of the thermoplastic synthetic resins having a lower fusion temperature.

4. The nonwoven fabric defined by any one of Claims 1 through 3, wherein the height dimension of the first crests is in a range of 1 to 5mm and the height dimension of the second crests is lower than the height dimension of the first crests by a range of 0.5 to 2mm.

5. The nonwoven fabric defined by any one of Claims 1 through 4, wherein the first crests and the second crests are formed alternately in the width direction and each of the troughs is interposed between each pair of the adjacent the first crest and the second crest.
